# FASCICULE DE BREVET EUROPEEN

(11) **EP 1 337 193 B1**
(45) Date de publication et mention de la délivrance du brevet: **20.06.2007**
(21) Numéro de dépôt: 01998274.3
(22) Date de dépôt: 30.11.2001
(51) Int. Cl.: A61B 17/70

(54) **DISPOSITIF DE STABILISATION INTERVERTEBRAL**
VORRICHTUNG ZUR ZWISCHENWIRBELSTABILITÄT
INTERVERTEBRAL STABILISING DEVICE

(30) Priorité: 01.12.2000 FR 0015621
(43) Date de publication de la demande: 27.08.2003
(73) Titulaire: Warsaw Orthopedic, Inc., Warsaw, IN 46581 (US)
(72) Inventeur: GRAF, Henry, 69006 Lyon (FR)
(74) Mandataire: Neyret, Daniel Jean Marie
(86) Numéro de dépôt international: PCT/FR2001/003804
(87) Numéro de publication internationale: WO 2002/043603

(56) Documents cités:
- WO-A-01/30248
- WO-A-01/39678
- WO-A-97/35529
- FR-A- 2 676 911
- US-A- 5 571 191

## Description

La présente invention concerne un dispositif de stabilisation intervertébral.

L'invention se propose de réaliser un tel dispositif, qui permet de restaurer la stabilité entre deux vertèbres adjacentes, lorsque l'articulation intervertébrale postérieure a été détruite, en tout ou partie, du fait de la chirurgie ou bien de la maladie.

A cet effet, elle a pour objet un dispositif de stabilisation intervertébral, destiné à relier deux vertèbres voisines, qui comprend un élément de butée supérieur, solidaire d'une vertèbre supérieure, ainsi qu'un élément de butée inférieur, solidaire d'une vertèbre inférieure, ces éléments de butée extra-discaux possédant des surfaces d'appui mutuelles, aptes à autoriser une rotation mutuelle desdites vertèbres supérieure et inférieure autour d'axes transversal et sagittal du patient, ainsi qu'à empêcher une rotation mutuelle de ces deux vertèbres autour d'un axe vertical, ces surfaces d'appui étant en outre aptes à autoriser une translation mutuelle de ces vertèbres dans un unique sens selon l'axe sagittal, à autoriser une translation entre ces deux vertèbres dans les deux sens selon l'axe vertical, et à interdire une translation entre ces deux vertèbres dans les deux sens selon l'axe transversal. US-A-557119 décrit une prothèse de l'articulation vertébrale postérieure apte à autoriser cette mobilité limitée. Le préambule de la revendication 1 est basé sur ce document. Selon l'invention, au moins une des surfaces d'appui a une forme sphérique.

Selon d'autres caractéristiques facultatives :
- l'un des éléments de butée comprend deux surfaces d'appui planes, disposées de part et d'autre de l'axe vertical, ces deux surfaces s'étendant de façon oblique et coopérant avec deux sphères dont est pourvu l'autre desdits éléments ;
- le dispositif comprend en outre au moins une vis pédiculaire supérieure, ainsi qu'au moins une vis pédiculaire inférieure, chaque élément de butée étant solidaire d'au moins une desdites vis pédiculaires ;
- chaque élément de butée est solidaire de deux vis pédiculaires, respectivement supérieures et inférieures ;
- il est prévu des moyens d'adaptation des dimensions transversales de chaque élément de butée, notamment au moins une lumière oblongue de réception d'une vis pédiculaire ;
- il est prévu des moyens permettant de solidariser en translation, de façon sélective, chaque élément de butée avec au moins une vis pédiculaire ;
- le dispositif comprend en outre un organe extradiscal, disposé à l'arrière de l'espace intervertébral, propre à amortir un déplacement entre lesdites vertèbres au moins dans le sens de la flexion intervertébral ;
- le dispositif comprend en outre au moins un implant intersomatique, destiné à être inséré au moins partiellement entre les corps vertébraux des deux vertèbres voisines.

L'invention va être décrite ci-dessous, en référence aux dessins annexés, donnés uniquement à titre d'exemples non limitatifs et dans lesquels :
- la figure 1 est une vue schématique de côté, illustrant deux vertèbres voisines entre lesquelles est placé un dispositif de stabilisation conforme à l'invention ;
- la figure 2 est une vue en perspective, illustrant le dispositif de la figure 1 ; et
- les figures 3 à 5 sont des vues de dessus, illustrant le dispositif de la figure 1, ainsi que deux variantes de réalisation.

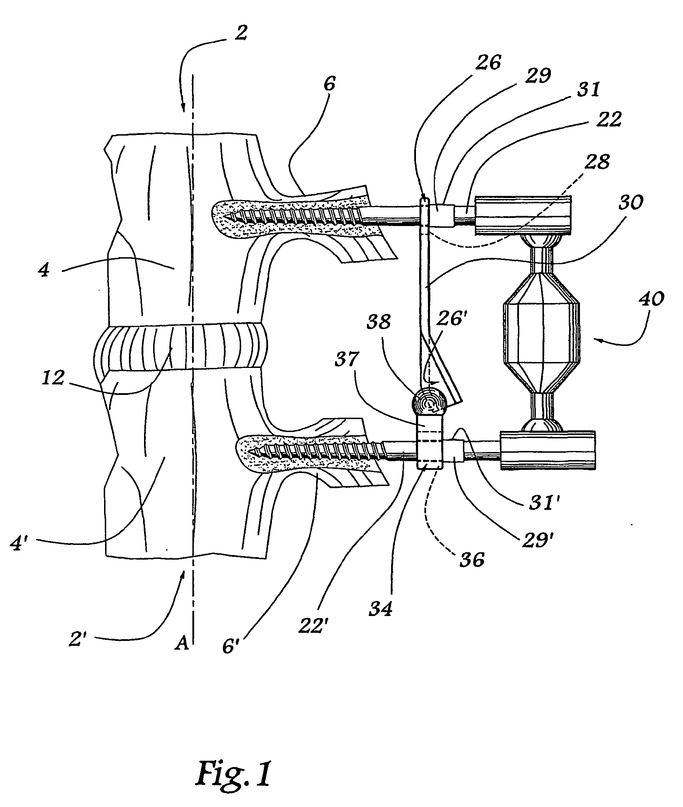

La figure 1 représente deux vertèbres respectivement supérieure 2 et inférieure 2', qui sont reliées par l'intermédiaire d'un dispositif de stabilisation conforme à l'invention. Chaque vertèbre comprend un corps vertébral 4, 4' prolongé par un pédicule 6, 6', et on désigne par 12 l'espace intervertébral. Il est à noter que le patient a subi une ablation d'une majeure partie de son articulation intervertébrale postérieure.

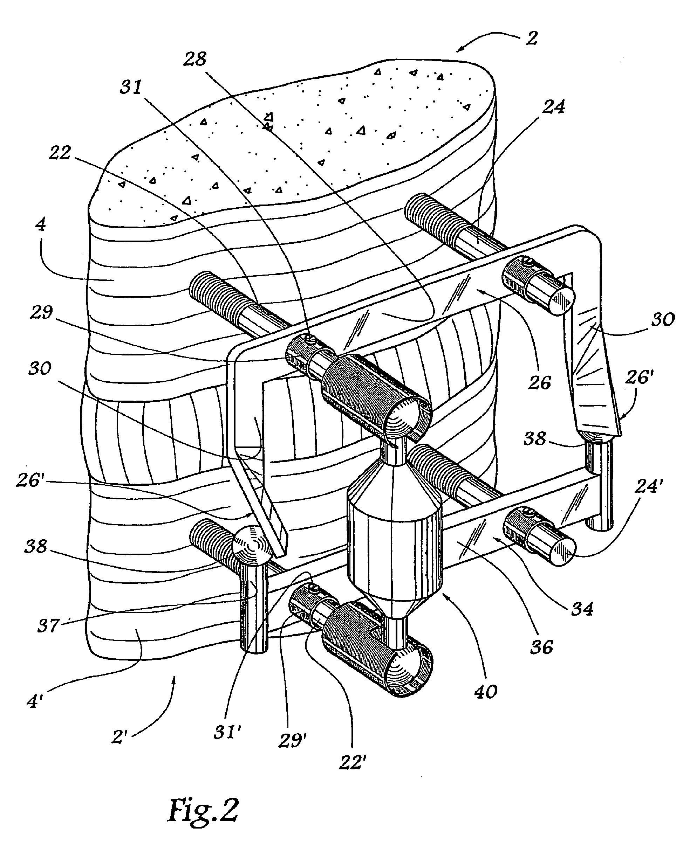

Comme le montre plus particulièrement la figure 2, la vertèbre supérieure est pourvue de deux vis pédiculaires supérieures 22, 24, disposées de part et d'autre de l'axe principal de la colonne vertébrale. Il est par ailleurs prévu deux vis pédiculaires inférieures 22', 24', solidaires de la vertèbre inférieure, qui sont sensiblement disposées à l'aplomb des vis pédiculaires supérieures.

Les dispositifs de stabilisation selon l'invention représentés sur les figures comprennent un élément de butée supérieur 26, comportant une branche horizontale 28 ainsi que deux branches verticales 30. Cette branche 28 est creusée de deux ouvertures circulaires destinées au passage de la tige des vis pédiculaires supérieures 22, 24. Les parois de chaque ouverture sont prolongées par un fourreau axial 29, recouvrant une partie de la vis. Ce fourreau, qui peut être venu de matière avec la branche 28, reçoit une vis d'arrêt 31 apte à immobiliser de façon sélective l'élément de butée par rapport à la vis pédiculaire, selon une translation parallèle à l'axe principal de cette dernière.

Ce dispositif comprend également un élément de butée inférieur 34, comportant une branche horizontale 36 prolongée, à ses extrémités, par des tiges 37 pourvues de sphères 38. Cet élément inférieur est creusé de deux ouvertures, destinées au passage de la tige des deux vis pédiculaires inférieures 22' , 24'. De façon analogue à ce qui a été décrit ci-dessus pour l'élément supérieur, chaque ouverture est pourvue d'un fourreau axial 29', muni d'une vis 31'.

Par ailleurs, en variante, au moins une des ouvertures peut être une lumière oblongue. Ceci permet ainsi d'adapter les dimensions transversales des éléments de butée à différents espacements des vis pédiculaires. Les branches horizontales 28 et 36 peuvent également présenter des longueurs variables, en étant par exemple télescopiques.

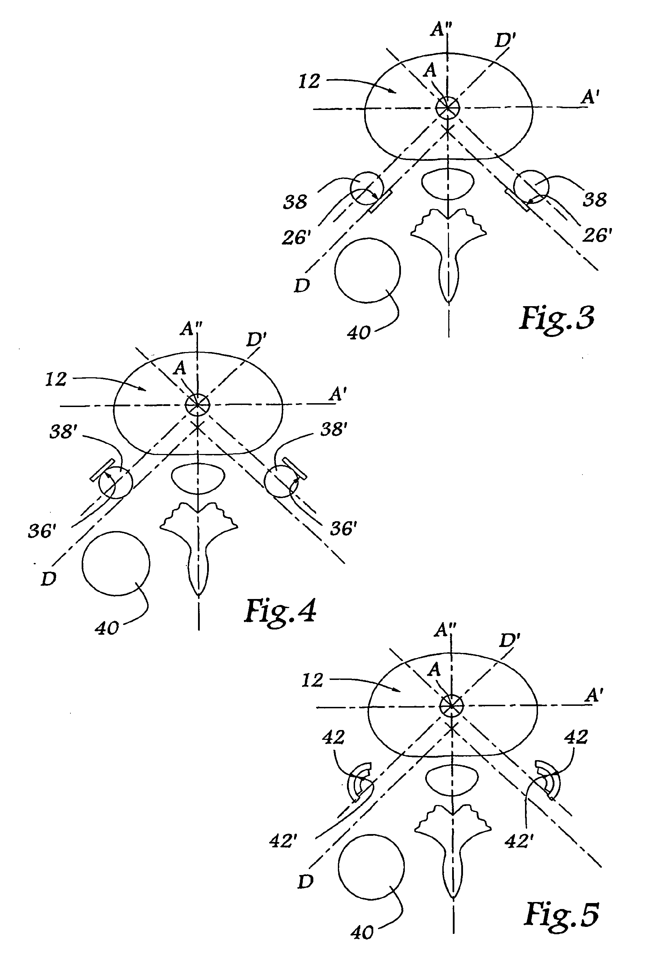

Chaque branche verticale 30 est repliée, de sorte que son extrémité possède une surface plane 26' s'étendant de façon oblique. Ceci signifie que cette extrémité n'est ni parallèle à l'axe transversal médian A', s'étendant de la droite vers la gauche du patient, ni parallèle à l'axe sagittal médian A", s'étendant d'arrière en avant du patient (figure 3). L'axe principal D de cette surface plane 26' est parallèle à une droite D' passant par l'intersection de ces deux axes A' et A", notamment une bissectrice de ces derniers.

Chaque surface d'appui 26' coopère avec une sphère 38 correspondante, selon un contact sensiblement ponctuel. De la sorte, deux rotations autour des axes A' et A" sont autorisées entre les éléments de butée supérieur et inférieur et, ce faisant, entre les deux vertèbres 2 et 2'. En revanche, la rotation autour de l'axe vertical A est interdite entre ces deux vertèbres.

Par ailleurs, une mise en translation mutuelle des deux vertèbres 2, 2', selon l'axe sagittal A", est autorisée, dans un unique sens. Ainsi, la vertèbre supérieure ne peut se déplacer vers l'avant, par rapport à la vertèbre inférieure, mais en revanche est libre de se déplacer vers l'arrière par rapport à cette vertèbre inférieure.

En outre, toute translation mutuelle des deux vertèbres 2, 2' est interdite, dans les deux sens, selon l'axe transversal A'. Enfin, une translation mutuelle entre ces deux vertèbres est autorisée, dans les deux sens, selon l'axe vertical A.

D'autres agencements peuvent être envisagés. Ainsi, l'élément de butée supérieur peut être muni d'au moins une sphère 38', coopérant avec une branche verticale, terminée par une surface plane oblique 36', s'étendant à partir de la branche horizontale 36 de l'élément inférieur (figure 4). Il peut être fait appel à la coopération de deux surfaces d'appui sphériques adjacentes 42, 42', dont chacune appartient à un élément de butée respectif (figure 5) .

A titre de variante supplémentaire, au moins une des branches verticales 30 peut, au moins partiellement, être réalisée en un matériau élastique, dont l'élasticité autorise un contact permanent entre chaque branche 30 et une sphère correspondante 38. Il est également envisageable de réaliser au moins une branche verticale en deux parties, possédant un certain débattement mutuel en rotation, autour de l'axe principal de la branche. Cette possibilité de débattement peut être provisoire, pour la mise en place des deux éléments de butée, ou permanente afin d'assurer à chaque instant une adaptation angulaire entre la branche et la sphère.

Il est possible de prévoir une unique branche verticale 30, coopérant avec une unique sphère 38, notamment dans le cas où une partie de l'articulation postérieure naturelle n'a pas été détruite.

Les deux vertèbres adjacentes 2, 2' sont reliées par ailleurs au moyen d'un organe d'amortissement 40, qui est fixé sur les deux extrémités libres des vis pédiculaires 22 et 22'. Cet organe d'amortissement est par exemple conforme à l'enseignement de FR-A-2 676 911, ou bien encore à celui de FR-A-2 751 864. Il peut également comprendre un ligament, conformément par exemple à l'enseignement de FR-A-2 694 182.

Cet organe d'amortissement extra-discal est propre à amortir un déplacement entre les deux vertèbres voisines au moins dans le sens de la flexion intervertébrale, dans laquelle le patient se penche vers l'avant.

L'invention n'est pas limitée aux exemples décrits et représentés.

On peut également prévoir de loger, dans l'espace intervertébral 12, un implant intersomatique, qui peut être partiel ou total. Dans le cas où il s'agit d'un implant partiel, plusieurs implants de ce type peuvent être disposés entre deux mêmes vertèbres.

Un tel implant peut être mis en place, soit par voie antérieure, soit par voie postérieure, par vissage ou encore par impaction.

L'invention permet de réaliser les objectifs précédemment mentionnés.

En cas de pathologie dégénérative du disque intervertébral, s'étendant aux nerfs qui lui sont adjacents, il est nécessaire pour le chirurgien de libérer la racine nerveuse ainsi comprimée. A cet effet, l'opération correspondante induit une destruction au moins partielle de l'articulation intervertébrale postérieure.

Le dispositif de l'invention permet de restaurer dans une mesure importante la stabilité postérieure, qui avait été sensiblement diminuée du fait de la chirurgie. En outre, il autorise un mouvement relatif entre les deux vertèbres voisines, qui est très proche du mouvement naturel. A cet égard, associer deux éléments de butée extra-discaux à un organe extra-discal d'amortissement est tout particulièrement avantageux.

Prévoir que chaque élément supérieur ou inférieur est monté sur deux vis pédiculaires à la fois permet d'éviter que ces vis ne se désolidarisent par rapport aux corps vertébraux qui les reçoivent. En effet, dans ce cas, les vis pédiculaires ne sont pas soumises à une quelconque rotation autour de leur axe principal.

## Revendications

1. Dispositif de stabilisation intervertébral, destiné à relier deux vertèbres voisines (2, 2'), qui comprend un élément de butée supérieur (26), solidaire d'une vertèbre supérieure (2), ainsi qu'un élément de butée inférieur (34), solidaire d'une vertèbre inférieure (2'), ces éléments de butée extra-discaux (26, 34) possédant des surfaces d'appui mutuelles (26', 38 ; 36', 38' ; 42, 42') aptes à autoriser une rotation mutuelle desdites vertèbres supérieure (2) et inférieure (2') autour d'axes transversal (A') et sagittal (A") du patient, ainsi qu'à empêcher une rotation mutuelle de ces deux vertèbres autour d'un axe vertical (A), ces surfaces d'appui étant en outre aptes à autoriser une translation mutuelle de ces vertèbres dans un unique sens selon l'axe sagittal (A"), à autoriser une translation entre ces deux vertèbres dans les deux sens selon l'axe vertical (A), et à interdire une translation entre ces deux vertèbres dans les deux sens selon l'axe transversal (A'), un premier (26, 34) desdits éléments de butée ayant au moins une surface d'appui sur un côté de l'axe vertical (A) et l'autre (34, 26) desdits éléments de butée ayant au moins une surface d'appui du même côté de l'axe vertical (A) qui coopère avec le premier élément de butée (26, 34), **caractérisé en ce qu'**au moins l'une desdites surfaces d'appui (38 ; 38' ; 42 ; 42') a une forme extérieure sphérique.

2. Dispositif selon la revendication 1, **caractérisé en ce que** chacun desdits éléments de butée (26, 34) comporte deux surfaces d'appui (26', 38 ; 36' ; 38' ; 42, 42') disposées de part et d'autre dudit axe vertical (A).

3. Dispositif selon la revendication 2, **caractérisé en ce que** l'un desdits éléments de butée comprend deux surfaces d'appui planes (26'), disposées de part et d'autre de l'axe vertical (A), ces deux surfaces s'étendant de façon oblique et coopérant avec deux sphères (38) dont est pourvu l'autre desdits éléments.

4. Dispositif selon la revendication 1 ou 2, **caractérisé en ce que** lesdites surfaces d'appui (42, 42') sont sphériques.

5. Dispositif selon l'une des revendications 1 à 4, **caractérisé en ce qu'**il comprend en outre au moins une vis pédiculaire supérieure (22, 24), ainsi qu'au moins une vis pédiculaire inférieure (22', 24'), chaque élément de butée (26, 34) étant solidaire d'au moins une desdites vis pédiculaires.

6. Dispositif selon la revendication 5, **caractérisé en ce que** chaque élément de butée (26, 34) est solidaire de deux vis pédiculaires, respectivement supérieures (22, 24) et inférieures (22', 24').

7. Dispositif selon la revendication 6, **caractérisé en ce qu'**il est prévu des moyens d'adaptation des dimensions transversales de chaque élément de butée, notamment au moins une lumière oblongue de réception d'une vis pédiculaire.

8. Dispositif selon l'une des revendications 5 à 7, **caractérisé en ce qu'**il est prévu des moyens (31, 31') permettant de solidariser en translation, de façon sélective, chaque élément de butée avec au moins une vis pédiculaire.

9. Dispositif selon l'une des revendications précédentes, **caractérisé en ce qu'**il comprend en outre un organe extradiscal (40), disposé à l'arrière de l'espace intervertébral (12), propre à amortir un déplacement entre lesdites vertèbres (2, 2') au moins dans le sens de la flexion intervertébral.

10. Dispositif selon l'une quelconque des revendications précédentes, **caractérisé en ce qu'**il comprend en outre au moins un implant intersomatique, destiné à être inséré au moins partiellement entre les corps vertébraux des deux vertèbres voisines.

## Claims

1. Intervertebral stabilising device designed to link two adjacent vertebrae (2, 2'), comprising an upper abutment element (26) rigidly connected to an upper vertebra (2) and a lower abutment element (34) rigidly connected to a lower vertebra (2'), said extradiscal abutment elements (26, 34) having mutual support surfaces (26', 38; 36', 38'; 42, 42') able to permit reciprocal rotation of said upper (2) and lower (2') vertebrae about transverse (A') and sagittal (A") axes of the patient and to prevent reciprocal rotation of said two vertebrae about a vertical axis (A), said support surfaces additionally being able to permit reciprocal translation of said vertebrae in a single direction of the sagittal axis (A"), to permit translation between said two vertebrae in both directions of the vertical axis (A) and to prevent translation between said two vertebrae in both directions of the transverse axis (A'), a first (26, 34) of said abutment elements having at least one support surface on one side of the vertical axis (A) and the other (34, 26) of said abutment elements having at least one support surface on the same side of the vertical axis (A), which latter support surface cooperates with the first abutment element (26, 34), **characterised in that** at least one of said support surfaces (38; 38'; 42; 42') has a spherical external form.

2. Device according to claim 1, **characterised in that** each of said abutment elements (26, 34) includes two support surfaces (26', 38; 36'; 38' 42, 42') disposed one on each side of said vertical axis (A).

3. Device according to claim 2, **characterised in that** one of said abutment elements includes two flat support surfaces (26') disposed one on each side of the vertical axis (A), said two surfaces extending obliquely and cooperating with two spheres (38) with which the other of said elements is provided.

4. Device according to claim 1 or 2, **characterised in that** said support surfaces (42, 42') are spherical.

5. Device according to any one of claims 1 to 4, **characterised in that** it additionally includes at least one upper pedicular screw (22, 24) and at least one lower pedicular screw (22', 24'), each abutment element (26, 34) being rigidly connected to at least one of said pedicular screws.

6. Device according to claim 5, **characterised in that** each abutment element (26, 34) is rigidly connected to two, respectively upper (22, 24) and lower (22', 24'), pedicular screws.

7. Device according to claim 6, **characterised in that** there are provided means for adapting the transverse dimensions of each abutment element, in particular at least one oblong opening for receiving a pedicular screw.

8. Device according to any one of claims 5 to 7, **characterised in that** there are provided means (31, 31') for enabling each abutment element having at least one pedicular screw to be selectively locked in translation.

9. Device according to any one of the preceding claims, **characterised in that** it additionally includes an extradiscal element (40) arranged behind the intervertebral space (12) and able to cushion displacement between said vertebrae (2, 2') at least in the direction of intervertebral deflection.

10. Device according to any one of the preceding claims, **characterised in that** it additionally includes at least one intersomatic implant designed to be inserted at least partially between the vertebral bodies of the two adjacent vertebrae.

## Patentansprüche

1. Vorrichtung zur intervertebralen Stabilisierung, die dazu dient, zwei benachbarte Wirbel (2,2') zu verbinden, enthaltend ein oberes Anschlagelement (26), das mit einem oberen Wirbel (2) verbunden ist, sowie ein unteres Anschlagelement (34), das mit einem unteren Wirbel (2') verbunden ist, wobei diese außerhalb der Bandscheibe liegenden Anschlagelemente (26,34), die gegenseitige Anschlagflächen (26',38;36',38';42,42') besitzen, geeignet sind, eine gegenseitige Rotation der oberen Wirbel (2) und unteren Wirbel (2') um Querachsen (A') und Sagittalachsen (A") des Patienten zu erlauben und eine gegenseitige Rotation dieser beiden Wirbel um eine Vertikalachse (A) zu verhindern, wobei die Anschlagflächen außerdem geeignet sind, eine gegenseitige Verschiebung dieser Wirbel in gleicher Richtung entlang der Sagittalachse (A") zu erlauben, eine Verschiebung zwischen diesen beiden Wirbeln in den beiden Richtungen entlang der Vertikalachse (A) zu erlauben und eine Verschiebung zwischen diesen beiden Wirbeln in den zwei Richtungen entlang der Querachse (A') zu verbieten, wobei ein erstes (26,34) der Anschlagelemente mindestens eine Anschlagfläche an einer Seite der Vertikalachse (A) und das andere (34,26) der Anschlagelemente mindestens eine Anschlagfläche auf derselben Seite der Vertikalachse (A) hat, die mit dem ersten Anschlagelement (26,34) zusammenwirkt, **dadurch gekennzeichnet, dass** mindestens eine der Anschlagflächen (38;38';42;42') eine kugelförmige äußere Form hat.

2. Vorrichtung nach Anspruch 1, **dadurch gekennzeichnet, dass** jedes der Anschlagelemente (26,34) zwei Anschlagflächen (26',38;36';38';42,42') aufweist, die zu beiden Seiten der Vertikalachse (A) angeordnet sind.

3. Vorrichtung nach Anspruch 2, **dadurch gekennzeichnet, dass** eines der Anschlagelemente zwei ebene Anschlagflächen (26') aufweist, die zu beiden Seiten der Vertikalachse (A) angeordnet sind, wobei diese beiden Flächen schief verlaufen und mit zwei Kugeln (38) zusammenwirken, mit denen das andere der Elemente versehen ist.

4. Vorrichtung nach Anspruch 1 oder 2, **dadurch gekennzeichnet, dass** die Anschlagflächen (42,42') kugelförmig sind.

5. Vorrichtung nach einem der Ansprüche 1 bis 4, **dadurch gekennzeichnet, dass** sie außerdem mindestens eine obere Stielschraube (22,24) sowie mindestens eine untere Stielschraube (22',24') aufweist, wobei jedes Anschlagelement (26,34) mit mindestens einer der Stielschrauben verbunden ist.

6. Vorrichtung nach Anspruch 5, **dadurch gekennzeichnet, dass** jedes Anschlagelement (26,34) mit zwei Stielschrauben, jeweils obere (22,24) und untere (22',24'), verbunden ist.

7. Vorrichtung nach Anspruch 6, **dadurch gekennzeichnet, dass** Mittel zur Anpassung der Abmessungen in Querrichtung jedes Anschlagelements vorgesehen sind, insbesondere mindestens eine längliche Öffnung zur Aufnahme einer Stielschraube.

8. Vorrichtung nach einem der Ansprüche 5 bis 7, **dadurch gekennzeichnet, dass** Mittel (31,31') vorgesehen sind, die es erlauben, in selektiver Weise jedes Anschlagelement mit mindestens einer Stielschraube beim Bewegen zu versteifen.

9. Vorrichtung nach einem der vorangehenden Ansprüche, **dadurch gekennzeichnet, dass** sie außerdem ein außerhalb der Bandscheibe angeordnetes Bauteil (40) aufweist, das hinter dem intervertebralen Raum (12) angeordnet ist und geeignet ist, eine Verschiebung zwischen den Wirbeln (2,2') mindestens in der Richtung der intervertebralen Biegung abzufedern.

10. Vorrichtung nach irgendeinem der vorangehenden Ansprüche, **dadurch gekennzeichnet, dass** sie außerdem mindestens ein intersomatisches Implantat aufweist, das dazu dient, zumindest teilweise zwischen den Wirbelkörpern der zwei benachbarten Wirbel eingesetzt zu werden.
